# EUROPEAN PATENT SPECIFICATION

(11) **EP 2 104 658 B1**
(45) Date of publication and mention of the grant of the patent: **09.12.2015**
(21) Application number: 07857108.0
(22) Date of filing: 21.12.2007
(51) Int. Cl.: C07C 29/141, C07C 29/145, C07H 3/00, C07H 3/04, C07H 1/00

(54) **A PROCESS FOR THE HYDROGENATION OF A SUGAR OR SUGAR MIXTURE**
VERFAHREN ZUR HYDRIERUNG EINES ZUCKERS ODER EINER ZUCKERMISCHUNG
PROCÉDÉ D'HYDROGÉNATION D'UN SUCRE OU D'UN MÉLANGE DE SUCRES

(30) Priority: 22.12.2006 EP 06026696; 22.12.2006 EP 06026697; 22.12.2006 EP 06026695
(43) Date of publication of application: 30.09.2009
(73) Proprietor: Cargill, Incorporated, Wayzata, MN 55391-5624 (US)
(72) Inventor: WEICHERT, Udo, 47906 Kempen (DE)
(74) Representative: Wibbelmann, Jobst
(86) International application number: PCT/EP2007/011402
(87) International publication number: WO 2008/077640

(56) References cited:
- EP-A1- 1 048 637
- GB-A- 742 553
- US-A- 3 329 729
- US-A- 5 644 044
- US-A- 5 679 781
- US-A- 5 936 081

## Description

The present invention relates to a process for the hydrogenation of a sugar or sugar mixture to produce a product mixture comprising at least two stereoisomeric sugar alcohols.

### Background of the invention

Keto-sugars (ketoses) or ketose-containing sugar mixtures (educts) are typically hydrogenated with hydrogen in an aqueous solution of the ketoses/ketose-containing mixtures at an elevated temperature and elevated pressure in the presence of a catalyst. This hydrogenation of the ketoses is usually resulting in a ratio of the corresponding sugar alcohol epimers of approximately 1:1.

With this type of educts it is possible to prepare mixtures of sugar alcohols wherein the ratio of the epimers is different from 1:1. There is a growing interest in improved sugar-free products comprising enriched mixtures of 1,1-GPM and 1,6-GPS. Applications of these enriched mixtures are disclosed in e.g. US 6,562,392.

It is therefore desirable to develop a process for certain applications, which produces reaction products with a predetermined ratio and eventually a ratio which is different from 1:1.

The prior art discloses various attempts to produce mixtures of the stereoisomers (epimers) wherein the ratio of said epimers is controlled (predefined).

Various documents disclose processes to produce sugar alcohols from sugars (e.g. WO 2006/063600 A1, DE 2520 173, US 3,912,804, US 6,020,472, Chen et al., Applied Catalysis A. General, 280 (2005), pp. 17-46, Albert et al., in: Moser: Catalysis of Organic Reactions (1978), pp. 421-439) at different temperatures and catalyst concentrations. However, typically the disclosed processes achieve an epimer ratio of approximately 1:1. In any event, the control of the ratio of said epimers is not addressed in these documents.

DE 2217 628 discloses the production of 1,6-GPS only by hydrogenation of isomaltulose when alkaline salts are added to the hydrogenation process so that the pH of the sugar solution is above 9 after the hydrogenation.

US 3,329,729 discloses the production of mannitol and sorbitol from an aqueous mixture of glucose and fructose under conditions where the pH value of the aqueous mixture is adjusted such that the hydrogenation is carried out under neutral, then alkaline and finally acidic conditions.

US 5,679,781 discloses the hydrogenation of isomaltulose using ruthenium and/or nickel as a catalyst at a pressure below 50 atmospheres. According to this disclosure, 1,1-GPM and 1,6-GPS are obtained in weight ratios of between 38:62 to 62:38, however, here too, the control of the obtained ratio is not addressed.

EP 1 048 637 A1 describes a two-step process converting glucosone into a product mixture containing mannitol and sorbitol at a ratio of alt least 3.5.

US 5,936,081 reports about a selective hydrogenation of isomaltulose to produce a mixture of 1-0-α-D-glucopyranosyl-D-mannitol (1,1-GPM) and 6-0-α-D-glucopyranosyl-D-sorbitol (1,6-GPS), occasionally referred to as mannitol and sorbitol epimers, respectively. As asserted in this document, the composition of the products (i.e. the ratio of the epimers) can be controlled by hydrogenating the isomaltulose under a predetermined stoichiometric ratio of hydrogen to educt. With an increase in the hydrogen/isomaltulose ratio, the mannitol/sorbitol epimer ratio shall be lowered. Accordingly, if the proportion of sorbitol epimer shall be high (thereby decreasing the mannitol/sorbitol epimer ratio) particular high pressures of hydrogen would be required and/or the flow of the educt isomaltulose has to be lowered. Both options are uneconomical.

Accordingly, there is a need in the prior art to adjust the stereo-selectivity of the hydrogenation reaction such that one of the stereoisomers is formed in greater proportions than the other stereoisomer. Thus, it is desirable to provide a process which produces reaction products with a predetermined ratio of the at least two stereoisomeric sugar alcohols (epimers) resulting from the hydrogenation of a ketose or a ketose-containing sugar mixture.

### The invention

The present invention suggests a process for the hydrogenation of a ketose or a ketose-containing sugar mixture to produce a product mixture comprising at least two stereoisomeric sugar alcohols, i.e. the cis-isomer and the trans-isomer sugar alcohol, comprising heating a reaction mixture comprising the ketose or ketose-containing sugar mixture in the presence of hydrogen with a particular predetermined concentration of a solid nickel-based catalyst, at a particular predetermined starting temperature and with a heating rate from the starting temperature (T₁) to an end temperature (T₂). In this hydrogenation reaction, the ratio of cis-isomer product to trans-isomer product can be controlled by i) varying the concentration of solid nickel-based catalyst, preferably Raney nickel, ii) varying the starting temperature of the heating step, and/or iii) varying the heating rate of the heating step.

More precisely, the ratio of cis-isomer to trans-isomer is decreased
i) by increasing the concentration of solid nickel-based catalyst, preferably Raney nickel,
ii) by decreasing the starting temperature of the heating step, and/or
iii) by decreasing the heating rate of the heating step.

Alternatively, the ratio of cis-isomer to trans-isomer is increased
i) by decreasing the concentration of solid nickel-based catalyst, preferably Raney nickel,
ii) by increasing the starting temperature of the heating step, and/or
iii) by increasing the heating rate of the heating step.

The cis-isomer refers to the cis-configuration of the hydroxyl groups at the C2-C3 carbon atom of the sugar alcohols, whereas the trans-isomer refers to the trans-configuration of the hydroxyl groups at the C2-C3 carbon atom of the sugar alcohols.

This process can be advantageously used in the hydrogenation of fructose, sorbose, erythrulose, lactulose, trehalulose, maltulose, isomaltulose, leucrose, fructooligosaccharides, and mixtures thereof. Preferably, isomaltulose, trehalulose, fructose, and mixtures thereof are hydrogenated. In a particular preferred embodiment, the process of the present invention utilizes a reaction mixture comprising isomaltulose (6-O-α-D-glucopyranosyl-D-fructose). Accordingly, the stereoisomeric sugar alcohols obtained by hydrogenation of an isomaltulose containing reactant mixture will contain 1-O-α-D-glucopyranosyl-D-mannitol (1,1-GPM, the cis-isomer) and 6-O-α-D-glucopyranosyl-D-sorbitol (1,6-GPS, the trans-isomer) at a controlled (predefined) ratio.

Isomaltulose is alpha-D-glucopyranosido-1,6-fructose and can be prepared from saccharose (sucrose) by enzymatic conversion using living or immobilized cell systems by known methods.

Trehalulose is referring to D-glucopyranosido-1,1-fructose. Lactulose is 4-beta-galactopyranosyl-D-fructose. Leucrose refers to 5-alpha-glucopyranosyl-fructose. Erythrulose is a tetrose carbohydrate with the chemical formula C₄H₈O₄, and also known as 1,3,4-trihydroxybutan-2-one.

The process of the invention can advantageously also be used for hydrogenating keto-sugar mixtures, such as the mixture of isomaltulose and trehalulose described in EP 625578.

In another preferred embodiment, the hydrogenation reaction mixture comprises fructose, resulting in the stereoisomeric sugar alcohols mannitol (cis-isomer) and sorbitol (trans-isomer) at a controlled (predefined) mannitol/sorbitol ratio.

Typically, the reaction mixture is an aqueous solution comprising the ketose or ketose-containing sugar mixture at a concentration of from 10% to 70% by weight (as dry matter). Preferably, the ketose or ketose-containing sugar mixture is present in the aqueous solution at a concentration of 15% to 60% by weight and more preferably from 30 to 50% by weight.

The hydrogenation may be carried out at a hydrogen pressure of between about 10 and 450 bar, preferably at 20 to 90 bar. The process may be carried out in a continuous, in a semi-continuous or in a batch mode.

In the process according to the present invention the ketoses or ketose-containing sugar mixtures are hydrogenated in the presence of hydrogen and a solid nickel-based catalyst, preferably a Raney nickel catalyst. These Raney catalysts can be used in various forms as described in the prior art (e.g., cf. Chen et al., Applied Catalysis A. General, 280 (2005), pp. 17-46, and references cited therein). With the process being generally carried out in the liquid phase, the mixture of catalyst with the remainder of the reaction mixture will be a heterogenous mixture. The solid nickel-based catalyst utilized in the context of the present invention is favourably employed at a concentration of 0,5% to 10% by weight, preferably between 1% and 8% by weight, more preferably between 2% and 7%, all based on the dry sugar content of the ketose or ketose-containing sugar mixture.

The inventor has surprisingly found that by keeping the reaction parameters the same but varying the concentration of the catalyst between different experiments, the epimer ratio can be shifted significantly. Hence, in one embodiment of the invention, the ratio of cis-isomer to trans-isomer is controlled by adjusting the concentration of the solid nickel-based catalyst. The predefined ratio of the epimers can be obtained by changing the catalyst concentration. The higher the catalyst concentration, the more trans-isomer and the less cis-isomer of the resulting sugar alcohol is obtained in the reaction mixture. The solid nickel-based catalyst concentration may be adjusted (at a given hydrogen pressure) for example
- by changing the catalyst amount for a given sugar solution volume and sugar solution concentration in a stirred batch process;
- by changing the sugar solution concentration for a given sugar solution volume and a given catalyst amount in a stirred batch process;
- by changing the sugar solution volume for a given sugar solution concentration and a given catalyst amount in a stirred batch process;
- by changing the sugar solution volume per hour rinsed over a given catalyst volume and a given sugar solution concentration;
- by changing the sugar solution concentration rinsed over a given catalyst volume and a given sugar solution volume per hour.

The two latter options can for example be applied in a continuous fixed bed reactor.

The current inventor further found, that the resulting epimer ratio can also be controlled by changing the starting temperature of the heating step. Therefore, a further embodiment of the present invention is characterized in that the starting temperature is adjusted in order to control the ratio of the at least two stereoisomeric sugar alcohols obtained in the hydrogenation reaction. By decreasing the starting temperature of the heating step, the ratio of cis-isomer to trans-isomer is decreased.

The reaction is carried out preferably at a temperature of between 20°C and about 150°C. The higher the starting temperature of the hydrogenation reaction, the more cis-isomer and the less trans-isomer of the resulting sugar alcohol are produced in the reaction. E.g. to obtain a particular epimer ratio, the starting temperature may be less than 40°C, such as about 30°C or about 20°C. Alternatively, the starting temperature may be within a range of 45 to 65°C, such as about 50°C, or it may be above 70°C.

The desired epimer ratio and, hence, the starting temperature of the hydrogenation reaction necessary to obtain this ratio can be provided in various ways, e.g.
- by feeding a sugar solution to a catalyst slurry under hydrogen pressure in a batch reactor at a specific starting temperature;
- by adding the catalyst to a sugar solution in a batch reactor, and subsequently pressurizing the reactor with hydrogen at a specific starting temperature; or
- by feeding continuously a sugar solution into a reactor containing a solid fixed bed catalyst at a specific starting temperature.

Further, the inventor has surprisingly found, that by keeping the reaction parameters the same but by varying the heating rate in different experiments, the epimer ratio can also be shifted. Therefore, one embodiment of the current invention is characterized in adjusting the heating rate to obtain the desired ratio of cis-isomer and trans-isomer of the obtained sugar alcohols. Hence, by decreasing the heating rate of the heating step, the ratio of cis-isomer to trans-isomer is decreased.

The reaction further comprises the heating of said reaction mixture which comprises a heating step from a starting temperature (T₁) to an end temperature (T₂) having a temperature difference T₂ - T₁ of at least 20°C or at least 30°C and which may span up to 130°C. E.g., this heating step could also be implemented by a gradual heating rate, such as a steady or a constant heating rate. A preferable heating rate is less than 6°C/min. In a more preferred embodiment of the invention, the heating rate is within a range of 0.5 to 5.0°C/min.

In the practice of the present invention, the starting temperature may be ambient temperature (such as 20°C; room temperature) and the end temperature is the desired temperature for finalizing the conversion/hydrogenation of the ketose or ketose-containing sugar mixture into the stereoisomeric sugar alcohols (e.g. 130°C). Preferably, the temperature difference T₂ - T₁ is the range of 40-70°C or higher. For instance, the reaction could be started at 50-60°C and stopped at an end temperature of 100-120°C. In a particular embodiment, it is preferred to conduct the process with a starting temperature of 20°C to 35°C to reach with a heating rate an end temperature of 120°C to 140°C.

In a preferred embodiment of the present invention, the process concerns the hydrogenation of a reaction mixture comprising isomaltulose so that the at least two isomeric sugar alcohols produced comprise 1,1-GPM and 1,6-GPS. With the processes according to the present invention a wide range of GPM/GPS ratios can be obtained from the same starting material by choosing the appropriate catalyst concentration while taking into account that the other reaction parameters such as heating rate and starting temperature are appropriately selected as well. Additionally or alternatively, a wide range of GPM/GPS ratios can also be obtained from the same starting material by choosing the appropriate heating rate and/or beginning the reaction at a suitable starting temperature, provided that the concentration of the catalyst is appropriately selected as well. In this way, e.g., GPM/GPS ratios between 0.6 and 1.2 or even between 0.55 and 1.25 can be achieved.

In a specific embodiment, the hydrogenation of 50% dry matter of isomaltulose is performed at 30 bar and at a starting temperature of 30°C, with a heating rate of 3°C/min and by increasing the catalyst concentration from 1 to 7%, the ratio of GPM/GPS (cis/trans isomer) is decreased from 0.96 to 0.78.

The ratio of cis/trans isomer is decreased from 0.83 to 0.68 while increasing the catalyst concentration from 1 to 7% while working at 130 bar with a 50% dry matter of isomaltulose at a starting temperature of 30°C and a heating rate of 3°C/min.

A further decrease of the ratio of cis/trans isomer is observed between 0.8 to 0.63 while increasing the catalyst concentration from 1 to 7% while working at 80 bar hydrogen gas pressure with a 30% dry matter of isomaltulose at a starting temperature of 30°C and a heating rate of 3°C/min.

In a further specific embodiment, the hydrogenation of 30% dry matter of isomaltulose is performed at 30 bar at a starting temperature of 20°C, in presence of 4% concentration of catalyst and by varying the heating rate from one experiment to the other from 0.5 to 4.6 °C/min the ratio of cis/trans isomers is increased from 0.61 to 0.74. A ratio as low as 0.57 can be achieved while bringing the heating rate to 0.5°C/min and at a hydrogen gas pressure of 130 bar. When working at a dry matter of isomaltulose of 50% and 80 bar, the increase of the heating rate form one experiment to the other from 0.5 to 4.3°C/min results in an increase of the ratio cis/trans isomer from 0.61 to 0.70.

In another embodiment, the hydrogenation of 30% dry matter of isomaltulose performed at 80 bar with a heating rate of 0.5 °C/min and increasing the starting temperature from one experiment to the other from 20°C to 80°C, increases the ratio of cis/trans isomer from 0.59 to 1.2.

Similarly to the described hydrogenation of isomaltulose, the obtained epimer ratios of hydrogenation reactions starting from other mono- and oligosaccharides can also be controlled by choosing a particular catalyst concentration, by choosing a particular starting temperature and/or by choosing a particular heating rate. Moreover, mixtures comprising considerable amounts of more than one ketose, such as solutions of isomaltulose and trehalulose, can be hydrogenated and the obtained stereoisomer ratio can be controlled according to the current invention, as illustrated in the Examples section.

A further embodiment of the present invention is the hydrogenation of fructose solutions. E.g., the hydrogenation of 30% dry matter of fructose is performed at 30 bar and at a starting temperature of 30°C, with a heating rate of 3°C/min and by increasing the catalyst concentration from 1 to 7%, the ratio of mannitol/sorbitol (cis/trans isomer) is decreased from 0.89 to 0.71.

In a further specific embodiment, the hydrogenation of 30% dry matter of fructose is performed at 30 bar at a starting temperature of 20°C, in presence of 4% concentration of catalyst and by varying the heating rate from one experiment to the other from 0.5 to 4.3 °C/min the ratio of cis/trans isomers is increased from 0.61 to 0.80.

Similarly, the obtained mannitol/sorbitol ratio can also be controlled by choosing a particular starting temperature and/or by choosing a particular heating rate of the reaction.

In general, the process according to the invention will be carried out in the liquid phase. In many cases, it may be appropriate to conduct the hydrogenation in the presence of a solvent. Basically, all common solvents can be used provided they are not degraded during the hydrogenation reactions. Examples of ordinary solvents include water, dioxane, tetrahydrofuran, DMSO, methanol, ethanol, n-propanol, isopropanol, n-butanol, cyclohexanol, ethylene glycol, 1,4-butane diol, 1,6-hexane diol, ethylene glycol methylether, and mixtures thereof, and the like. In the context of the present invention, however, the use of water or water-containing reaction mixtures is preferred.

After hydrogenation, the polyol solutions are usually stripped off the catalyst. In a batch process, this can be effected first by sedimentation of the catalyst. Subsequently, the remaining catalyst is removed from the supernatant by filtration, centrifugation or magnetic separation means as completely as possible. Of course, the first sedimentation step does not apply when packed bed catalysts are used. In a second step, solved components, as e.g. salts, organic acids, proteins, etc. are removed by ion exchange processes. For this purpose, the solution is treated with cation exchange resins of weak and/or strong acidity and with anion exchange resins of weak and/or strong basicity. Moreover, undesired contaminations such as colour, smell and/or taste components can further be removed by adsorption on activated carbon, polisher resins, polymers, etc. Finally, the desired dry matter content is adjusted by vaporization of the solvent.

The sugar alcohols produced in the process according to the invention are typically crystallized, solidified or liquid syrups (concentrated liquid syrups containing dry substance of 50% or higher). These solidified, crystallized or liquid syrup products or the immediate reactions products of the process can be used in the production of various products. Thus, these product mixtures can be advantageously used for the coating of products in the sweetener or pharmaceutical industry. Moreover, candies, in particular hard candies, may be prepared from the liquid or viscous product of the process according to the present invention which is cooked at a temperature of between 145°C and 170°C (optionally in the presence of a low vacuum) followed by a conventional shaping of the final candy (having finally a water content of less than 4% by weight, preferably less than 2% by weight, based on the weight of the candy).

Other objects and features of the present invention will become apparent from the following detailed description. All % values in the following are given in % by weight unless indicated otherwise.

### Examples and Detailed Description of Preferred Embodiments

### Examples 1-3

The following embodiments of the present invention relate to a process for the preparation of a mixture of the stereoisomeric sugar alcohols 6-O-α-D-glucopyranosyl-D-sorbitol (1,6-GPS) and 1-O-α-D-glucopyranosyl-D-mannitol (1,1-GPM) by hydrogenation of isomaltulose using a Raney nickel catalyst, wherein the ratio of 1,6-GPS/1,1-GPM can be regulated by adjusting the catalyst concentration.

Isomaltulose was hydrogenated in a pressurizable hydrogenation reactor with an inner volume of 1,000 ml (without internal fixtures) equipped with an inductively operated mixer, further containing a hollow shaft with gassing stirrer, a sampling valve with a sintered metal frit for sampling the reaction solution and a PT 100 temperature probe. Mixing was performed at 1,220 rpm. The isomaltulose reagent used has the composition shown in Table 1.

**Table 1**

| **Ingredient** | **Content,** as dry matter |
|---|---|
| Isomaltulose | 98.9% |
| Dextrose | < 0.1% |
| Fructose | < 0.1% |
| Isomaltose | < 0.1% |
| Trehalulose | 0.4% |
| Others | 0.5% |

The composition of this raw material was analysed by ion chromatography and HPLC. The amounts of isomaltulose, dextrose, fructose, isomaltose and trehalulose (and sucrose, if present) were determined on a CarboPac PA1 column (Dionex GmbH, Idstein, Germany); the amount of "others", which is mainly oligosaccharides, was determined on a classical Ca²⁺-form cation exchanger, such as Aminex HPX 87 C (Bio-Rad Laboratories, Munich, Germany).

The composition of the obtained hydrogenation product samples were determined on a classical Ca²⁺-form cation exchanger, such as Aminex HPX 87 C (Bio-Rad Laboratories, Munich, Germany), except for isomaltulose, which was quantified on a CarboPac PA1 column (Dionex GmbH, Idstein, Germany), if the amount of isomaltulose was below 2% by weight on dry base. Sucrose was determined by an enzymatic test (Art.Nr. 10 716 260 035, R-Biopharm, Darmstadt, Germany).

### Example 1

### Hydrogenation of isomaltulose solution, 50% dry matter, 30 bar

816 g aqueous isomaltulose solution (50% dry matter) was introduced into the autoclave reactor. At a temperature of 30°C, the solution was supplemented with 4.1 g, 16.3 g and 28.6 g of nickel catalyst (Degussa, BK 111 W), respectively, corresponding to 1%, 4% and 7% of catalyst, respectively, based on the dry sugar content of the isomaltulose solution. The reactor was closed and inertized 3 times with nitrogen. The nitrogen was exchanged with hydrogen 3 times and subsequently, the hydrogen pressure was adjusted to 30 bar. The rotation speed was increased to 1,220 rpm and the isomaltulose solution was heated from 30°C to 130°C with a heating rate of 3°C/min. Consumed hydrogen was automatically replenished so that the pressure of 30 bar was maintained throughout the course of the hydrogenation reaction. Samples were taken when the temperature of 130°C was reached, having the compositions set out in Table 2.

**Table 2**

| | | | |
|---|---|---|---|
| Catalyst concentration [% of dry sugar] | 1 | 4 | 7 |
| Heating rate [°C/min] | 3 | 3 | 3 |
| GPS* [% of dry sugar] | 48.3 | 53.9 | 55.5 |
| 1,1-GPM [% of dry sugar] | 46.5 | 45.1 | 43.6 |
| Sorbitol [% of dry sugar] | 0.1 | 0.1 | 0.1 |
| Mannitol [% of dry sugar] | 0.1 | 0.1 | 0.1 |
| Isomaltulose [% of dry sugar] | 3.5 | <0.05 | <0.05 |
| Others [% of dry sugar] | 1.5 | 0.9 | 0.8 |
| GPM/GPS ratio | 0.963 | 0.837 | 0.786 |

| | | | |
|---|---|---|---|
| GPS*: GPS consists primarily of 1,6-GPS and minor amounts of 1,1-GPS, the latter being formed from trehalulose. 1,1-GPS and 1,6-GPS co-elute in the HPLC method used. | | | |

It is evident from Table 2 that the GPM/GPS ratio is dependant on the catalyst concentration at constant heating rate. The ratio cis/trans isomer (GPM/GPS) decreases with increased catalyst concentration

The reaction containing 1% catalyst was continued for an additional hour at 130°C in order to demonstrate that the reaction can be completed and the remaining isomaltulose content can be reduced below 0.05% without changing the obtained GPM/GPS ratio significantly.

### Example 2

### Hydrogenation of isomaltulose solution, 50% dry matter, 130 bar

816 g aqueous isomaltulose solution (50% dry matter) was introduced into the autoclave reactor. At a temperature of 30°C the solution was supplemented with 4.1 g, 16.3 g and 28.6 g of nickel catalyst (Degussa, BK 111 W), respectively, corresponding to 1%, 4% and 7% of catalyst, respectively, based on the dry sugar content of the isomaltulose solution. The reactor was closed and inertized 3 times with nitrogen. The nitrogen was exchanged with hydrogen 3 times and subsequently, the hydrogen pressure was adjusted to 130 bar. The rotation speed was increased to 1,220 rpm and the isomaltulose solution was heated from 30°C to 130°C with a heating rate of 3°C/min. Consumed hydrogen was automatically replenished so that the pressure of 130 bar was maintained throughout the course of the hydrogenation reaction. Samples were taken when the temperature of 130°C was reached, having the compositions set out in Table 3.

**Table 3**

| | | | |
|---|---|---|---|
| Catalyst concentration [% of dry sugar] | 1 | 4 | 7 |
| Heating rate [°C/min] | 3 | 3 | 3 |
| GPS [% of dry sugar] | 53.8 | 57.6 | 58.9 |
| 1,1-GPM [% of dry sugar] | 45.0 | 41.6 | 40.3 |
| Sorbitol [% of dry sugar] | 0.1 | 0.1 | 0.1 |
| Mannitol [% of dry sugar] | 0.1 | 0.1 | 0.1 |
| Isomaltulose [% of dry sugar] | <0.05 | <0.05 | <0.05 |
| Others [% of dry sugar] | 1.0 | 0.7 | 0.6 |
| GPM/GPS ratio | 0.836 | 0.722 | 0.684 |

It is evident from Table 3 that the GPM/GPS ratio is dependant on the catalyst concentration at constant heating rate. The ratio cis/trans isomer (GPM/GPS) decreases with increased catalyst concentration.

### Example 3

### Hydrogenation of isomaltulose solution, 30% dry matter, 80 bar

746 g aqueous isomaltulose solution (30% dry matter) was introduced into the autoclave reactor. At a temperature of 30°C the solution was supplemented with 2.2 g, 9.0 g and 15.7 g of nickel catalyst (Degussa, BK 111 W), respectively, corresponding to 1%, 4% and 7% of catalyst, respectively, based on the dry sugar content of the isomaltulose solution. The reactor was closed an inertized 3 times with nitrogen. The nitrogen was exchanged with hydrogen 3 times and subsequently, the hydrogen pressure was adjusted to 80 bar. The rotation speed was increased to 1,220 rpm and the isomaltulose solution was heated from 30°C to 130°C with a heating rate of 3°C/min. Consumed hydrogen was automatically replenished so that the pressure of 80 bar was maintained throughout the course of the hydrogenation reaction. The samples were taken when the temperature of 130°C was reached, having the compositions set out in table 4.

**Table 4**

| | | | |
|---|---|---|---|
| Catalyst concentration [% of dry sugar] | 1 | 4 | 7 |
| Heating rate [°C/min] | 3 | 3 | 3 |
| GPS [% of dry sugar] | 54.6 | 58.7 | 60.7 |
| 1,1-GPM [% of dry sugar] | 44.1 | 40.5 | 38.6 |
| Sorbitol [% of dry sugar] | 0.1 | 0.1 | 0.1 |
| Mannitol [% of dry sugar] | 0.1 | <0.05 | 0.1 |
| Isomaltulose [% of dry sugar] | 0.1 | <0.05 | <0.05 |
| Others [% of dry sugar] | 1.1 | 0.6 | 0.5 |
| GPM/GPS ratio | 0.808 | 0.690 | 0.636 |

It is evident from table 4 that the GPM/GPS ratio is dependent on the catalyst concentration at constant heating rate. The ratio cis/trans isomer (GPM/GPS) decreases with increased catalyst concentration.

### Example 4

### Hydrogenation of isomaltulose/trehalulose solution, 30% dry matter, 30 bar

**Table 5**

| **Ingredient** | **Content,** as dry matter |
|---|---|
| Isomaltulose | 85.8 % |
| Dextrose | 1.5 % |
| Fructose | 2.6 % |
| Isomaltose | 0.5 % |
| Trehalulose | 8.0 % |
| Sucrose | 0.1 % |
| Others | 1.5 % |

746 g aqueous isomaltulose/trehalulose solution (30% dry matter) of the composition shown in Table 5 (quantification according to the method explained for Examples 1-4) was introduced into the autoclave reactor. At a temperature of 30°C the solution was supplemented with 2.2 g, 9.0 g and 15.7 g of nickel catalyst (Degussa, BK 111 W), respectively, corresponding to 1%, 4% and 7% of catalyst, respectively, based on the dry sugar content of the isomaltulose/trehalulose solution. The reactor was closed and inertized 3 times with nitrogen. The nitrogen was exchanged with hydrogen 3 times and subsequently, the hydrogen pressure was adjusted to 30 bar. The rotation speed was increased to 1,220 rpm and the isomaltulose/trehalose mixture was heated from 30°C to 130°C with a heating rate of 3°C/min. Consumed hydrogen was automatically replenished so that the pressure of 30 bar was maintained throughout the course of the hydrogenation reaction. Samples were taken when the temperature of 130°C was reached having the compositions set out in Table 6 (quantification according to the method explained for Examples 1-3).

**Table 6**

| | | | |
|---|---|---|---|
| Catalyst concentration [% of dry sugar] | 1 | 4 | 7 |
| Heating rate [°C/min] | 3 | 3 | 3 |
| GPS [% of dry sugar] | 44.8 | 52.4 | 54.4 |
| 1,1-GPM [% of dry sugar] | 40.5 | 41.3 | 39.4 |
| Sorbitol [% of dry sugar] | 2.1 | 3.1 | 3.1 |
| Mannitol [% of dry sugar] | 1.2 | 1.2 | 1.2 |
| Isomaltulose [% of dry sugar] | 5.5 | <0.05 | <0.05 |
| Others [% of dry sugar] | 6.0 | 1.9 | 1.9 |
| GPM/GPS- ratio | 0.904 | 0.788 | 0.724 |

It is evident from Table 6 that the GPM/GPS ratio is dependant on the catalyst concentration at constant heating rate. The ratio cis/trans isomer (GPM/GPS) decreases with increased catalyst concentration.

The reaction containing 1% catalyst was continued for an additional hour at 130°C in order to demonstrate that the reaction can be completed and the remaining isomaltulose content can be reduced below 0.05% without changing the obtained GPM/GPS ratio significantly.

### Example 5

### Hydrogenation of fructose solution, 30% dry matter, 30 bar

751 g aqueous fructose solution (30% dry matter, composition: 99.7% fructose; 0.2% dextrose; 0.1% others; the composition was analysed by HPLC using a classical Ca²⁺-form cation exchanger, such as Aminex HPX 87 C (Bio-Rad Laboratories, Munich, Germany)) was introduced into the autoclave reactor. At a temperature of 30°C the solution was supplemented with 2.3 g, 9.0 g and 15.8 g of nickel catalyst (Degussa, BK 111 W), respectively, corresponding to 1%, 4% and 7% of catalyst, respectively, based on the dry sugar content of the fructose solution. The reactor was closed and inertized 3 times with nitrogen. The nitrogen was exchanged with hydrogen 3 times and subsequently the hydrogen pressure was adjusted to 30 bar. The rotation speed was increased to 1,220 rpm and the fructose mixture was heated from 30°C to 130°C with a heating rate of 3°C/min. Consumed hydrogen was automatically replenished so that the pressure of 30 bar was maintained throughout the course of the hydrogenation reaction. Samples were taken when the temperature of 130°C was reached, having the compositions set out in Table 7.

The composition of the hydrogenation product compositions were determined on a classical Ca²⁺-form cation exchanger, such as Aminex HPX 87 C (Bio-Rad Laboratories, Munich, Germany), except for fructose, which was determined by an enzymatic test (Art.Nr. 10 716 260 035, R-Biopharm, Darmstadt, Germany), if the amount of fructose was below 1% by weight on dry base.

**Table 7**

| | | | |
|---|---|---|---|
| Catalyst concentration [% of dry sugar] | 1 | 4 | 7 |
| Heating rate [°C/min] | 3 | 3 | 3 |
| D-Sorbitol [% of dry sugar] | 47.1 | 56.5 | 58.2 |
| D-Mannitol [% of dry sugar] | 42.3 | 43.1 | 41.5 |
| L-Iditol [% of dry sugar] | <0.05 | 0.1 | 0.1 |
| D-Fructose [% of dry sugar] | 9.8 | <0.05 | <0.05 |
| Others[% of dry sugar] | 0.8 | 0.3 | 0.2 |
| Mannitol/Sorbitol ratio | 0.898 | 0.763 | 0.713 |

It is evident from Table 7 that the mannitol/sorbitol ratio is dependant on the catalyst concentration, too, at a constant heating rate. The ratio cis/trans isomer (mannitol/sorbitol) decreases with increased catalyst concentration.

The reaction containing 1% catalyst was continued for an additional hour at 130°C in order to demonstrate that the reaction can be completed and the remaining fructose content can be reduced below 0.05% without changing the obtained mannitol/ sorbitol ratio significantly.

These data demonstrate that the dependency of the isomer ratio obtained by hydrogenation on the catalyst concentration is a general principle observable with different reactants.

### Examples 6-8

The following embodiments of the present invention relate to a process for the preparation of a mixture of the stereoisomeric sugar alcohols 6-O-α-D-glucopyranosyl-D-sorbitol (1,6-GPS) and 1-O-α-D-glucopyranosyl-D-mannitol (1,1-GPM) by hydrogenation of isomaltulose using a Raney nickel catalyst at increased hydrogen pressure, wherein the ratio of 1,6-GPS/1,1-GPM can be regulated by changing the heating rate of the reaction mixture. The isomaltulose reagent is specified in Table 1 (supra). The isomaltulose was hydrogenated as described for Examples 1-3 with different starting temperatures as mentioned in the following examples. For analysis of the obtained hydrogenation product samples, the same procedures were utilized as stated above.

### Example 6

### Hydrogenation of isomaltulose solution, 30% dry matter, 30 bar

746 g aqueous isomaltulose solution (30% dry matter), introduced into the autoclave reactor, was supplemented with 9.0 g nickel catalyst (Degussa, BK 111 W), corresponding to 4% of catalyst based on the dry matter content of the isomaltulose solution. The reactor was closed and inertized twice with nitrogen. The nitrogen was exchanged with hydrogen twice and subsequently, the hydrogen pressure was adjusted to 30 bar while mixing at 333 rpm. The rotation speed was increased to 1,220 rpm and the isomaltulose solution was heated from about 20°C to 130°C in different experiments with a heating rate of 0.5°C/min, 1.0°C/min, 2.7°C/min or 4.6°C/min, respectively. Consumed hydrogen was automatically replenished so that the pressure of 30 bar was maintained throughout the course of the hydrogenation reaction. In each experiment, a sample was taken when the temperature of 130°C was reached, having the composition set out in Table 8.

**Table 8**

| | | | | |
|---|---|---|---|---|
| Starting temperature [°C] | 20 | 20 | 20 | 20 |
| End temperature [°C] | 130 | 130 | 130 | 130 |
| Heating rate [°C/min] | 0.5 | 1.0 | 2.7 | 4.6 |
| GPS [% of dry matter] | 61.4 | 60.4 | 58.2 | 56.9 |
| 1,1-GPM [% of dry matter] | 37.8 | 38.9 | 41.0 | 42.2 |
| Sorbitol [% of dry matter] | 0.1 | 0.1 | 0.1 | 0.1 |
| Mannitol [% of dry matter] | <0.05 | <0.05 | <0.05 | <0.05 |
| Isomaltulose [% of dry matter | <0.05 | <0.05 | <0.05 | <0.05 |
| Others [% bez. TS] | 0.6 | 0.6 | 0.7 | 0.8 |
| GPM/GPS-ratio | 0.616 | 0.644 | 0.704 | 0.742 |

It is evident from Table 8 that the GPM/GPS ratio is dependant on the heating rate of the reaction mixture. The ratio cis/trans isomer (GPM/GPS) increases with increased heating rate.

### Example 7

### Hydrogenation of isomaltulose solution, 30% dry matter, 80 and 130 bar

Reactions similar to Example 6 were performed with various heating rates under different hydrogen pressures (see Table 9). Apart from adjusting the hydrogen pressure to 80 bar and 130 bar, respectively, and maintaining these pressures throughout the reaction, the conditions described above remained unaltered.

The obtained GPM/GPS ratios are summarized in Table 9.

**Table 9**

| Heating rate [°C/min] | Isomaltulose (80 bar) | Isomaltulose (130 bar) |
|---|---|---|
| 0.5 | 0.587 | 0.576 |
| 1.0 | 0.609 | 0.590 |

### Example 8

### Hydrogenation of isomaltulose solution, 50% dry matter, various pressures

816 g aqueous isomaltulose solution (50% dry matter), introduced into the autoclave reactor, was supplemented with 16.3 g nickel catalyst (Degussa, BK 111 W), corresponding to 4% of catalyst based on the dry matter content of the isomaltulose solution. The reactor was closed and inertized twice with nitrogen. The nitrogen was exchanged with hydrogen twice and subsequently, the hydrogen pressure was adjusted to 80 bar while mixing at 333 rpm. The rotation speed was increased to 1,220 rpm and the isomaltulose solution was heated from about 20°C to 130°C at various heating rates, respectively. Consumed hydrogen was automatically replenished so that the pressure of 80 bar was maintained throughout the course of the hydrogenation reaction. Samples were taken when the temperature of 130°C was reached, having the compositions set out in Table 10.

**Table 10**

| | | | | |
|---|---|---|---|---|
| Hydrogen pressure [bar] | 80 | 80 | 80 | 80 |
| Starting temperature [°C] | 20 | 20 | 20 | 20 |
| End temperature [°C] | 130 | 130 | 130 | 130 |
| Heating rate [°C/min] | 0.5 | 1.0 | 2.9 | 4.3 |
| GPS [% of dry matter] | 61.5 | 60.8 | 59.2 | 58.1 |
| 1,1-GPM [% of dry matter] | 37.8 | 38.5 | 40.0 | 41.0 |
| Sorbitol [% of dry matter] | 0.1 | 0.1 | 0.1 | 0.1 |
| Mannitol [% of dry matter] | <0.05 | <0.05 | 0.1 | 0.1 |
| Isomaltulose [% of dry matter] | <0.05 | <0.05 | <0.05 | <0.05 |
| Others [% of dry matter] | 0.6 | 0.6 | 0.7 | 0.8 |
| GPM/GPS-ratio | 0.615 | 0.633 | 0.676 | 0.706 |

It is evident from Table 10 that the GPM/GPS ratio is dependent on the heating rate of the reaction mixture.

Similar experiments were performed at hydrogen pressures of 30 bar and 130 bar, respectively. These results (GPM/GPS ratio) are summarized in Table 11.

**Table 11**

| Heating rate [°C/min] | Isomaltulose (30 bar) | Isomaltulose (80 bar) | Isomaltulose (130 bar) |
|---|---|---|---|
| 0.5 | 0.650 | 0.615 | 0.602 |
| 1.0 | 0.673 | 0.633 | 0.620 |
| 2.9 | 0.754 | 0.676 | 0.650 |
| 4.3 | | 0.706 | |
| 4.4 | | | 0.672 |
| 4.6 | 0.810 | | |

These results demonstrate that the dependency on the heating rate is maintained over a large range of H₂ pressures applied.

### Example 9

### Hydrogenation of isomaltulose solution, 30% dry matter, 80 bar with a starting temperature of 80°C

746 g aqueous isomaltulose solution (30% dry matter) was introduced into the autoclave reactor and heated to 80°C while stirring. 9.0 g nickel catalyst (Degussa, BK 111 W) was added, corresponding to 4% of catalyst based on the dry matter content of the isomaltulose solution. The reactor was closed and inertized twice with nitrogen. The nitrogen was exchanged with hydrogen twice and subsequently, the hydrogen pressure was adjusted to 80 bar. During the reaction, the solution was stirred with 1,220 rpm and the temperature was raised to 130°C at various heating rates, respectively. Consumed hydrogen was automatically replenished so that the pressure of 80 bar was maintained throughout the course of the hydrogenation reaction. Samples were taken when the temperature of 130°C was reached, having the compositions set out in Table 12.

**Table 12**

| | | |
|---|---|---|
| Starting temperature [°C] | 80 | 80 |
| End temperature [°C] | 130 | 130 |
| Heating rate [°C/min) | 0.5 | 1.0 |
| GPS [% of dry matter] | 45.7 | 45.1 |
| 1,1-GPM [% of dry matter] | 52.9 | 53.3 |
| Sorbitol [% of dry matter] | 0.1 | 0.1 |
| Mannitol [% of dry matter] | 0.1 | 0.1 |
| Isomaltulose [% of dry matter] | <0.05 | <0.05 |
| Others [% of dry matter] | 1.2 | 1.4 |
| GPM/GPS-ratio | 1.158 | 1.182 |

### Example 10

### Hydrogenation of fructose solution, 30% dry matter, 30 bar

751 g aqueous fructose solution (30% dry matter, composition: 99.7% fructose; 0.2% dextrose; 0.1% others; the composition was analysed by HPLC using a classical Ca²⁺-form cation exchanger, such as Aminex HPX 87 C (Bio-Rad Laboratories, Munich, Germany)), introduced into the autoclave reactor described above, was supplemented with 9.0 g nickel catalyst (Degussa, BK 111 W), corresponding to 4% of catalyst based on the dry matter content of the fructose solution. The reactor was closed and inertized twice with nitrogen. The nitrogen was exchanged with hydrogen twice and subsequently, the hydrogen pressure was adjusted to 30 bar while mixing at 333 rpm. The rotation speed was increased to 1220 rpm and the fructose solution was heated from about 20°C to 130°C with various heating rates, respectively. Consumed hydrogen was automatically replenished so that the pressure of 30 bar was maintained throughout the course of the hydrogenation reaction. Samples were taken when the temperature of 130°C was reached, having the compositions set out in Table 13.

The composition of the hydrogenation product compositions were determined on a classical Ca²⁺-form cation exchanger, such as Aminex HPX 87 C (Bio-Rad Laboratories, Munich, Germany), except for fructose, which was determined by an enzymatic test (Art.Nr. 10 716 260 035, R-Biopharm, Darmstadt, Germany), if the amount of fructose was below 1% by weight on dry base.

**Table 13**

| | | | | |
|---|---|---|---|---|
| Starting temperature [°C] | 20 | 20 | 20 | 20 |
| End temperature [°C] | 130 | 130 | 130 | 130 |
| Heating rate [°C/min] | 0.5 | 1.0 | 2.9 | 4.3 |
| D-Sorbitol [% of dry matter] | 61.7 | 60.2 | 57.0 | 55.1 |
| D-Marinitol [% of dry matter] | 37.9 | 39.4 | 42.5 | 44.0 |
| L-Iditol [% of dry matter] | 0.2 | 0.2 | 0.1 | 0.1 |
| D-Fructose [% of dry matter] | <0.05 | <0.05 | <0.05 | 0.2 |
| Others [% of dry matter] | 0.2 | 0.3 | 0.4 | 0.5 |
| Mannitol/Sorbitol-ratio | 0.614 | 0.654 | 0.746 | 0.799 |

It is evident from Table 13 that the mannitol/sorbitol ratio is dependant on the heating rate of the reaction mixture. The ratio cis/trans isomer (mannitol/sorbitol) increases with increased heating rate.

### Example 11

### Hydrogenation of isomaltulose/trehalulose solution, 30% dry matter, 30 bar

746 g aqueous isomaltulose/trehalulose solution (30% dry matter) of a composition shown in Table 6 (, see Example 5; quantification according to the method explained for Examples 1-4), introduced into the autoclave reactor described above, was supplemented with 9.0 g nickel catalyst (Degussa, BK 111 W), corresponding to 4% of catalyst based on the dry matter content of the isomaltulose/trehalulose solution. The reactor was closed and inertized twice with nitrogen. The nitrogen was exchanged with hydrogen twice and subsequently, the hydrogen pressure was adjusted to 30 bar while mixing at 333 rpm. The rotation speed was increased to 1,220 rpm and the isomaltulose/ trehalulose solution was heated from about 20°C to 130°C with various heating rates, respectively. Consumed hydrogen was automatically replenished so that the pressure of 30 bar was maintained during the course of the hydrogenation reaction. Samples were taken when the temperature of 130°C was reached, having the compositions set out in Table 14 (quantification according to the method explained for Examples 1-3).

**Table 14**

| | | | | |
|---|---|---|---|---|
| Starting temperature [°C] | 20 | 20 | 20 | 20 |
| End temperature [°C] | 130 | 130 | 130 | 130 |
| Heating rate [°C/min] | 0.5 | 1.0 | 3.0 | 4.7 |
| GPS [% of dry matter] | 56.7 | 55.7 | 53.7 | 52.5 |
| 1,1-GPM [% of dry matter] | 37.1 | 38.2 | 40.1 | 41.1 |
| Sorbitol [% of dry matter] | 3.2 | 3.1 | 3.1 | 3.0 |
| Mannitol [% of dry matter] | 1.1 | 1.2 | 1.2 | 1.2 |
| Isomaltulose [% of dry matter] | <0.05 | <0.05 | <0.05 | <0.05 |
| Sucrose [% of dry matter] | 0.1 | 0.1 | 0.1 | 0.1 |
| Others [% of dry matter] | 1.8 | 1.7 | 1.8 | 2.1 |
| GPM/GPS-ratio | 0.654 | 0.686 | 0.747 | 0.783 |

It is evident from Table 14 that the GPM/GPS ratio is dependant on the heating rate of the reaction mixture.

The data of the last two examples further demonstrate that the dependency of the isomer ratio obtained by hydrogenation on the heating rate is a general principle observable with different reactants and reactant mixtures.

## Claims

1. A process for the controlled hydrogenation of a ketose or a ketose-containing sugar mixture to produce a product mixture comprising at least two stereoisomeric sugar alcohols in a predefined ratio of cis-isomer to trans-isomer, comprising
a) providing a reaction mixture comprising said ketose or ketose-containing sugar mixture,
b) adding to said reaction mixture a concentration of solid nickel-based catalyst based upon the dry sugar content of the ketose or the ketose-containing sugar mixture, preferably a concentration of Raney nickel, and
c) conducting in said reaction mixture a hydrogenation reaction in presence of hydrogen gas,
**characterized in that** the process is comprising a heating step and wherein the ratio of cis-isomer to trans-isomer is decreased either
i) by increasing the concentration of solid nickel-based catalyst, preferably Raney nickel,
ii) by decreasing the starting temperature of the heating step, and/or
iii) by decreasing the heating rate of the heating step.

2. The process according to claim 1 wherein the ketose or ketose-containing sugar mixture is selected from the group consisting of fructose, sorbose, erythrulose, lactulose, trehalulose, maltulose, isomaltulose, leucrose, fructooligosaccharides, and mixture thereof, preferably isomaltulose, trehalulose, fructose, and mixtures thereof.

3. The process according to claim 1 or 2 wherein the reaction mixture is an aqueous solution comprising the ketose or ketose-containing sugar mixture at a concentration of from 10% to 70% by weight.

4. The process according to any one of the preceding claims wherein the hydrogenation is carried out at a hydrogen pressure between 10 and 450 bar.

5. The process according to any one of the preceding claims wherein the concentration of solid nickel-based catalyst is between 0.5% and 10% by weight, preferably between 2% and 7% by weight, based on the dry sugar content of the ketose or a ketose-containing sugar mixture.

6. The process according to any one of the preceding claims wherein the heating rate is less than 6°C/min.

7. The process according to any one of claims 1-5 wherein the heating rate is in the range of 0.5 to 5.0°C/min.

8. The process according to any one of the preceding claims wherein the heating step is implemented by a heating rate from a starting temperature (T₁) to an end temperature (T₂) with a temperature difference T₂ - T₁ of at least 20°C, preferably at least 30°C.

9. The process according to any one of the preceding claims wherein the hydrogenation reaction is carried out at a temperature of between 20°C and 150°C.

10. The process according to any one of claims 1-8 wherein the hydrogenation reaction is carried out at a temperature of between 30°C and 130°C, preferably 90°C.

11. The process according to any one of the preceding claims wherein the reaction mixture comprises isomaltulose such that said at least two stereoisomeric sugar alcohols comprise 1-O-α-D-glucopyranosyl-D-mannitol (1,1-GPM) and 6-O-α-D-glucopyranosyl-D-sorbitol (1,6-GPS).

12. The process according to any one of claims 1-10 wherein the reaction mixture comprises fructose such that said at least two stereoisomeric sugar alcohols comprise mannitol and sorbitol.

## Patentansprüche

1. Verfahren zur kontrollierten Hydrierung einer Ketose oder eines ketosehaltigen Zuckergemischs zum Herstellen eines Produktgemischs, das mindestens zwei stereoisomere Zuckeralkohole in einem vordefinierten Verhältnis von Cis-Isomer zu Trans-Isomer umfasst, das Folgendes umfasst:
a) Bereitstellen eines Reaktionsgemisches, das die Ketose oder das ketosehaltige Zuckergemisch umfasst,
b) Beimengen einer Konzentration von festem Katalysator auf Nickelbasis zu dem Reaktionsgemisch basierend auf dem Trockenzuckergehalt der Ketose oder des ketosehaltigen Zuckergemisches, vorzugsweise einer Konzentration von Raney-Nickel, und
c) Durchführen einer Hydrierungsreaktion in dem Reaktionsgemisch bei Vorhandensein von Wasserstoffgas,
**dadurch gekennzeichnet, dass** das Verfahren einen Erhitzungsschritt umfasst und wobei das Verhältnis von Cis-Isomer zu Trans-Isomer vermindert wird entweder:
i) durch Erhöhen der Konzentration an festem Katalysator auf Nickelbasis, vorzugsweise Raney-Nickel,
ii) durch Verringern der Ausgangstemperatur des Erwärmungsschrittes und/oder
iii) durch Verringern der Aufheizgeschwindigkeit des Erhitzungsschrittes.

2. Verfahren nach Anspruch 1, wobei die Ketose oder das ketosehaltige Zuckergemisch von der Gruppe ausgewählt wird, die aus Fruktose, Sorbose, Erythrulose, Lactulose, Trehalulose, Maltulose, Isomaltulose, Leucrose, Fructooligosacchariden und Gemischen davon, vorzugsweise Isomaltulose, Trehalulose, Fruktose und Gemischen davon, besteht.

3. Verfahren nach Anspruch 1 oder 2, wobei das Reaktionsgemisch eine wässrige Lösung ist, die die Ketose oder das ketosehaltige Zuckergemisch mit einer Konzentration von 10 bis 70 Gew.-% umfasst.

4. Verfahren nach einem der vorhergehenden Ansprüche, wobei die Hydrierung bei einem Wasserstoffdruck zwischen 10 und 450 bar durchgeführt wird.

5. Verfahren nach einem der vorhergehenden Ansprüche, wobei die Konzentration an festem Katalysator auf Nickelbasis zwischen 0.5 und 10 Gew.-%, vorzugsweise zwischen 2 und 7 Gew.-%, basierend auf dem Trockenzuckergehalt der Ketose oder eines ketosehaltigen Zuckergemisches beträgt.

6. Verfahren nach einem der vorhergehenden Ansprüche, wobei die Aufheizgeschwindigkeit niedriger als 6 °C/min ist.

7. Verfahren nach einem der Ansprüche 1 bis 5, wobei die Aufheizgeschwindigkeit im Bereich von 0.5 bis 5.0 °C/min liegt.

8. Verfahren nach einem der vorhergehenden Ansprüche, wobei der Erhitzungsschritt durch eine Aufheizgeschwindigkeit von einer Ausgangstemperatur (T₁) zu einer Endtemperatur (T₂) mit einer Temperaturdifferenz T₂ - T₁ von mindestens 20 °C, vorzugsweise mindestens 30 °C, ausgeführt wird.

9. Verfahren nach einem der vorhergehenden Ansprüche, wobei die Hydrierungsreaktion bei einer Temperatur zwischen 20 °C und 150 °C durchgeführt wird.

10. Verfahren nach einem der Ansprüche 1 bis 8, wobei die Hydrierungsreaktion bei einer Temperatur zwischen 30 °C und 130 °C, vorzugsweise 90 °C, durchgeführt wird.

11. Verfahren nach einem der vorhergehenden Ansprüche, wobei das Reaktionsgemisch Isomaltulose umfasst, derart, dass die mindestens zwei stereoisomeren Zuckeralkohole 1-O-α-D-Glocopyranosyl-D-Mannitol (1,1-GPM) und 6-O-α-D-Glocopyranosyl-D-Sorbitol (1,6-GPS) umfassen.

12. Verfahren nach einem der Ansprüche 1 bis 10, wobei das Rektionsgemisch Fruktose umfasst, derart, dass die mindestens zwei stereoisomeren Zuckeralkohole Mannitol und Sorbitol umfassen.

## Revendications

1. Procédé d'hydrogénation contrôlée d'une cétose ou d'un mélange de sucres contenant une cétose pour produire un mélange de produits comprenant au moins deux alcools glucidiques stéréoisomères dans un rapport prédéfini de cis-isomère au trans-isomère, comprenant
a) l'apport d'un mélange réactionnel comprenant ladite cétose ou ledit mélange de sucres contenant une cétose,
b) l'ajout audit mélange réactionnel d'une concentration de catalyseur à base de nickel solide basée sur la teneur en sucre sec de la cétose ou du mélange de sucres contenant une cétose, de préférence d'une concentration de nickel de Raney, et
c) la réalisation dans ledit mélange réactionnel d'une réaction d'hydrogénation en présence de gaz d'hydrogène,
**caractérisé en ce que** le procédé comprend une étape de chauffage et dans lequel le rapport du cis-isomère au trans-isomère est réduit soit
i) en augmentant la concentration de catalyseur à base de nickel solide, de préférence de nickel de Raney,
ii) en réduisant la température de départ de l'étape de chauffage, et/ou
iii) en réduisant la vitesse de chauffage de l'étape de chauffage.

2. Procédé selon la revendication 1 dans lequel la cétose ou le mélange de sucres contenant une cétose est choisi(e) dans le groupe constitué du fructose, du sorbose, de l'érythrulose, du lactulose, du tréhalulose, du maltulose, de l'isomaltulose, du leucrose, des fructooligosaccharides et de leur mélange, de préférence de l'isomaltulose, du tréhalulose, du fructose et de leurs mélanges.

3. Procédé selon la revendication 1 ou 2 dans lequel le mélange réactionnel est une solution aqueuse comprenant la cétose ou le mélange de sucres contenant une cétose à une concentration de 10 % à 70 % en poids.

4. Procédé selon l'une quelconque des revendications précédentes dans lequel l'hydrogénation est réalisée à une pression d'hydrogène située entre 10 et 450 bar.

5. Procédé selon l'une quelconque des revendications précédentes dans lequel la concentration de catalyseur à base de nickel solide se situe entre 0,5 % et 10 % en poids, de préférence entre 2 % et 7 % en poids, sur la base de la teneur en sucre sec de la cétose ou d'un mélange de sucres contenant une cétose.

6. Procédé selon l'une quelconque des revendications précédentes dans lequel la vitesse de chauffage est inférieure à 6 °C/min.

7. Procédé selon l'une quelconque des revendications 1 à 5 dans lequel la vitesse de chauffage se situe dans la plage de 0,5 à 5,0 °C/min.

8. Procédé selon l'une quelconque des revendications précédentes dans lequel l'étape de chauffage est mise en oeuvre par une vitesse de chauffage d'une température de départ (T₁) à une température finale (T₂) avec une différence de température T₂ - T₁ d'au moins 20 °C, de préférence d'au moins 30 °C.

9. Procédé selon l'une quelconque des revendications précédentes dans lequel la réaction d'hydrogénation est réalisée à une température située entre 20 °C et 150 °C.

10. Procédé selon l'une quelconque des revendications 1 à 8 dans lequel la réaction d'hydrogénation est réalisée à une température située entre 30 °C et 130 °C, de préférence égale à 90 °C.

11. Procédé selon l'une quelconque des revendications précédentes dans lequel le mélange réactionnel comprend de l'isomaltulose de manière à ce que lesdits au moins deux alcools glucidiques stéréoisomères comprennent du 1-O-α-D-glucopyranosyl-D-mannitol (1,1-GPM) et du 6-O-α-D-glucopyranosyl-D-sorbitol (1,6-GPS).

12. Procédé selon l'une quelconque des revendications 1 à 10 dans lequel le mélange réactionnel comprend du fructose de manière à ce que lesdits au moins deux alcools glucidiques stéréoisomères comprennent du mannitol et du sorbitol.
